# FASCICULE DE BREVET EUROPEEN

(11) **EP 1 019 510 B1**
(45) Date de publication et mention de la délivrance du brevet: **16.07.2003**
(21) Numéro de dépôt: 98946547.1
(22) Date de dépôt: 01.10.1998
(51) Int. Cl.: C12N 15/34, C07K 14/01, A61K 39/12, A61K 48/00, C12Q 1/68, C07K 16/08, G01N 33/53

(54) **VACCINS A BASE DE CIRCOVIRUS PORCINS**
SCHWEINECIRCOVIREN ABGELEITETE IMPFSTOFFE
VACCINES DERIVED FROM PORCINE CIRCOVIRUSES

(30) Priorité: 03.10.1997 FR 9712382; 22.01.1998 FR 9800873; 20.03.1998 FR 9803707
(43) Date de publication de la demande: 19.07.2000
(60) Demande divisionnaire: 02017134.4
(73) Titulaire: MERIAL, 69002 Lyon (FR); The Queen's University of Belfast, Belfast BT4 3SD (GB); The University of Saskatchewan, Saskatoon, Saskatchewan S7W 5B4 (CA)
(72) Inventeur: ALLAN, Gordon, Belfast BT5 7AQ (GB); MEEHAN, Brian, Belfast BT1 3LX (GB); CLARK, Edward, Saskatoon, Saskatchewan S7J 214 (CA); ELLIS, John, Saskatoon, Saskatchewan S7N 0M3 (CA); HAINES, Deborah, Saskatoon, Saskatchewan SN7 0M3 (CA); HASSARD, Lori, Saskatoon, Saskatchewan S7K 2A0 (CA); HARDING, John, Humboldt, Saskatchewan 2 S0K 2A0 (CA); CHARREYRE, Catherine, Elisabeth, F-69720 Saint-Laurent de Mure (FR); CHAPPUIS, Gilles, Emile, F-69006 Lyon (FR); MCNEILLY, Francis, Newtownards, BT3 4NH (GB)
(74) Mandataire: Colombet, Alain André
(86) Numéro de dépôt international: FR9802107
(87) Numéro de publication internationale: WO99018214

(56) Documents cités:
- WO-A-96/06619
- NAYAR G.P.S. ET AL.: "Detection and characterization of porcine circovirus associated with postweaning multisystemic wasting syndrome in pigs" CANADIAN VETERINARY JOURNAL - REVUE VETERINAIRE CANADIENNE, vol. 38, juin 1997, pages 385-386, XP002068396 cité dans la demande
- CLARK E. G.: "Post-weaning multisystemic wasting syndrome" PROCEEDINGS OF THE AMERICAN ASSOCIATION OF SWINE PRACTITIONERS. ANNUAL MEETING, 1 mars 1997, pages 499-501, XP002068397 cité dans la demande
- MEEHAN B.M. ET AL.: "Characterization of novel circovirus DNAs associated with wasting syndromes in pigs" JOURNAL OF GENERAL VIROLOGY, vol. 79, no. 9, septembre 1998, pages 2171-2179, XP002090386
- HAMEL A.L. ET AL.: "Nucleotide sequence of Porcine Circovirus associated with postweaning multisystemic wasting syndrome in pigs" JOURNAL OF VIROLOGY, vol. 72, no. 6, juin 1998, pages 5262-5267, XP002078783
- MOROZOV I. ET AL.: "Detection of a novel strain of porcine circovirus in pigs with postweaning multisystemic wasting syndrome" JOURNAL OF CLINICAL MICROBIOLOGY, vol. 36, no. 9, septembre 1998, pages 2535-2541, XP002090921
- MEEHAN B.M. ET AL.: "Sequence of porcine circovirus DNA: affinities with plant circoviruses" JOURNAL OF GENERAL VIROLOGY, vol. 78, no. 1, janvier 1997, pages 221-227, XP002068398 cité dans la demande
- MANKERTZ A. ET AL.: "Mapping and characterization of the origin of DNA replication of porcine circovirus" JOURNAL OF VIROLOGY, vol. 71, no. 3, mars 1997, pages 2562-2566, XP002078782 cité dans la demande

## Description

La présente invention est relative à un vaccin à base de nouvelles souches de circovirus porcin (PCV pour *Porcine CircoVirus*) responsables du syndrome PMWS *(Porcine Multisystemic Wasting Syndrome* ou *Post-Weaning Multisystemic Wasting Syndrome* encore appelé syndrome de dépérissement généralisé de post-sevrage).

Le PCV a été à l'origine détecté comme contaminant non cytopathogène dans des lignées cellulaires de reins de porcs PK/15. Ce virus a été classé parmi les Circoviridae avec le virus de l'anémie du poulet (CAV pour *Chicken Anemia Virus*) et le virus PBFDV (*Pscittacine Beak and Feather Disease Virus).* Il s'agit de petits virus (de 15 à 24 nm) non enveloppés dont la caractéristique commune est de contenir un génome sous forme d'un ADN simple brin circulaire de 1,76 à 2,31 kb. On a d'abord pensé que ce génome codait pour un polypeptide d'environ 30 kDa (Todd et al., Arch Virol 1991, 117: 129-135). Des travaux récents ont toutefois montré une transcription plus complexe (Meehan B. M. et al., 1997, 78: 221-227). Par ailleurs, on ne connaît pas d'homologies significatives de séquence nucléotidique ni de déterminants antigéniques communs entre les trois types de circovirus connus.

Le PCV issu des cellules PK/15 est considéré comme n'étant pas pathogène. On en connaît la séquence d'après B. M. Meehan et al., J Gen Virol 1997 (78) 221-227. Ce n'est que très récemment que des auteurs ont pensé que des souches de PCV pourraient être pathogènes et associées au syndrome PMWS (Gupi P. S. Nayar et al., Can Vet J , vol. 38, 1997: 385-387 et Clark E. G., Proc Am Assoc Swine Prac 1997: 499-501). Nayar et al. ont détecté de l'ADN de PCV chez des porcs présentant le syndrome PMWS par des techniques de PCR. Aucune souche sauvage de PCV n'a toutefois été isolée et purifiée à ce jour.

Le syndrome PMWS détecté au Canada, aux Etats-Unis et en France se caractérise au plan clinique par une perte progressive de poids et par des manifestations telles que tachypnée, dyspnée et jaunisse. Au plan pathologique, il se traduit par des infiltrations lymphocytaires ou granulomateuses, des lymphadénopathies et, plus rarement, par des hépatites et néphrites lymphocytaires ou granulomateuses (Clark E. G., Proc. Am. Assoc. Swine Prac. 1997: 499-501 ; La Semaine Vétérinaire n° 26, supplément à La Semaine Vétérinaire 1996 (834) ; La Semaine Vétérinaire 1997 (857): 54 ; Gupi P. S. Nayar et al., Can Vet J , vol. 38, 1997: 385-387).

La déposante a réussi à isoler cinq souches nouvelles de PCV à partir de prélèvements pulmonaires ou ganglionnaires provenant d'élevages situés au Canada, aux Etats-Unis (Californie) et en France (Bretagne), ci-après dénommés circovirus selon l'invention. Ces virus ont été mis en évidence dans des lésions de porcs atteints du syndrome PMWS, mais pas chez des porcs sains.

La déposante a en outre séquencé le génome de quatre de ces souches, à savoir les souches provenant du Canada et des Etats-Unis ainsi que deux souches française. Les souches présentent entre elles une très forte homologie au niveau nucléotidique dépassant 96 % et beaucoup plus faible avec la souche PK/15, environ 76 %. Les nouvelles souches peuvent donc être considérées comme représentatives d'un nouveau type de circovirus porcin, dénommé ici type II, le type I étant représenté par la PK/15.

La présente invention permet d'obtenir le circovirus porcin de groupe II, tel que défini ci-dessus, isolé ou sous forme de préparation purifiée.

La présente invention a pour objet un vaccin induisant une réponse immunitaire contre le circovirus porcin de type II (PCV type II), comprenant un circovirus porcin de type II responsable du syndrome PMWS, sous forme inactivée.

L'invention concerne en particulier tout circovirus porcin susceptible d'être isolé d'un échantillon physiologique ou d'un prélèvement tissulaire, notamment de lésions, d'un porc malade présentant le syndrôme PMWS, notamment en suivant la méthode décrite dans les exemples, en particulier circovirus du type II.

La présente invention a plus particulièrement pour objet l'utilisation des préparations purifiées de cinq souches, qui ont été déposées auprès de l'ECACC (European Collection of Cell Cultures, Centre for Applied Microbiology & Research, Porton Down, Salisbury, Wiltshire SP4 OJG, Royaume-Uni) le jeudi 2 octobre 1997:
- n° d'accès V97100219 (appelé ici Imp.1008PCV)
- n° d'accès V97100218 (appelé ici lmp.1010PCV)
- n° d'accès V97100217 (appelé ici lmp.999PCV).
   et, le vendredi16 janvier1998 :
- n° d'accès V98 011608 (appelé ici lmp 1011-48285)
- n° d'accès V98 011609 (appelé ici lmp 1011-48121)

L'invention entend considérer les circovirus porcins isolés d'un porc malade et/ou les circovirus ayant une parenté sérologique significative avec les souches de l'invention et/ou les circovirus ayant une hybridation croisée avec les souches de l'invention dans des conditions de stringence telles qu'il n'y a pas d'hybridation avec la souche PCV PK/15,

Les souches virales isolées d'un échantillon physiologique ou d'un prélèvement tissulaire, notamment d'une lésion, d'un porc présentant le syndrome PMWS peuvent être avantageusement propagées sur des lignées cellulaires telles que notamment des lignées cellulaires de rein de porc, en particulier cellules PK/15 indemnes de contamination (en particulier pour PCV, ainsi que pour pestivirus, adénovirus porcin et parvovirus porcin) en vue de leur multiplication ou spécifiquement pour la production d'antigène, entier (e.g. virus) et/ou sous-unités (e.g. polypeptides).

De manière très remarquable et inattendue, ces isolats se sont révélés très productifs en culture sur cellules PK/15, ce qui présente des avantages indéniables pour la production de virus ou d'antigène, pour la production de vaccin inactivé.

La présente invention permet donc d'obtenir des préparations de circovirus isolés après passages sur cellules, notamment lignées cellulaires, e.g. cellules PK/15, cultivées in vitro en étant infectées par l'un au moins des circovirus selon l'invention ou de tout circovirus porcin susceptible d'être isolé d'un échantillon physiologique ou d'un prélèvement tissulaire, notamment de lésions, d'un porc présentant le syndrome PMWS. Elle a aussi pour objet les surnageants ou extraits de culture, éventuellement purifiés par des techniques standards, et de manière générale toute préparation antigénique obtenue à partir des cultures in vitro.

Les vaccins comprennent en outre un véhicule ou diluant acceptable sur le plan vétérinaire, avec éventuellement en plus un adjuvant acceptable sur le plan vétérinaire.

Les circovirus selon l'invention, avec les fractions qui peuvent être présentes, sont inactivés selon les techniques connues de l'homme du métier. L'inactivation sera effectuée de préférence par voie chimique, e.g. par exposition de l'antigène à un agent chimique tel que formaldéhyde (formol), paraformaldéhyde, β-propiolactone ou éthytène imine ou ses dérivés. La méthode d'inactivation préférée sera ici l'exposition à un agent chimique et en particulier à l'éthylène imine ou à la β-propiolactone.

De préférence, les vaccins inactivés selon l'invention seront adjuvés, avantageusement en étant présentés sous forme d'émulsions, par exempte eau-dans-l'huile ou huile-dans-l'eau, selon les techniques bien connues de l'homme du métier. Le caractère adjuvant pourra aussi provenir de l'incorporation au principe actif d'un composé adjuvant usuel.

Parmi les adjuvants qui peuvent être utilisés, on peut citer à titre d'exemple l'hydroxyde d'alumine, les saponines (e.g. Quiliaja saponin ou Quit A ; voir Vaccine Design, The Subunit and Adjuvant Approach, 1995, édité par Michael F. Powel et Mark J. Newman, Plennum Press, New-York and London, p 210), l'Avridine® (Vaccine Design p 148), le DDA (Diméthyldioctadécylammonium bromide, Vaccine Design p 157), le Polyphosphazene (Vaccine Design p 204), ou encore des émulsions huile dans-l'eau à base d'huile minérale, de squalane (e.g. émulsion SPT, Vaccine Design p 147), de squalène (e.g. MF59, Vaccine Design p 183), ou eau-dans-l'huile à base d'huile métabolisable (de préférence selon WO-A-94 20071) ainsi que les émulsions décrites dans US-A-5 422 109. On peut aussi choisir des associations d'adjuvants, par exemple Avridine® ou DDA associé à une émulsion.

Ces vaccins comprendront de préférence de 10⁶ à 10⁸ TCID50.

La déposante a en outre obtenu le génome de quatre des isolats, identifiés SEQ ID NO: 1 à 4 et éventuellement 6.

La présente invention donne donc accès à un fragment d'ADN contenant tout ou partie de l'une de ces séquences. Il va de soi que l'invention recouvre automatiquement les séquences équivalentes, c'est-à-dire les séquences ne changeant pas la fonctionnalité ni la spécificité de souche de la séquence décrite ni des polypeptides codés par cette séquence. Seront bien entendu incluses les séquences différant par dégénérescence du code.

L'invention recouvre également les séquences équivalentes en ce sens qu'elles sont capables de s'hybrider à la séquence supra dans des conditions de stringence élevées et/ou ont une forte homologie avec les souches de l'invention et appartiennent au groupe II défini plus haut.

Les vaccins inactivés selon l'invention pourront comprendre un ou des principes actifs (antigènes) d'un ou de plusieurs (2 ou 3) des circovirus selon l'invention.

L'invention prévoit aussi d'associer la vaccination contre le circovirus porcin à une vaccination contre d'autres pathogènes du porc, en particulier ceux pouvant être associés au syndrome PMWS. Les vaccins inactivés selon l'invention pourront donc comprendre une autre valence correspondant à un autre pathogène du porc. Parmi ces autres pathogènes du porc, on peut citer de préférence le PRRS (Porcine Reproductory and Respiratory Syndrome) (l'homme du métier pourra se reporter à WO-A-93/07898, WO-A-94/18311, FR-A-2 709 966 ; C. Chareyre et al., Proceedings of the 15th IPVS Congress Birmingham, England, 5-9 juillet 1998, p 139 ; incorporés par référence) et/ou Mycoplasma hyopneumoniae (l'homme du métier pourra se reporter à EP-A-597 852, EP-A-550 477, EP-A-571 648, O. Martinon et al., p 167, p 284, p 285 et G. Reynaud et al., p 150 du Proceedings of the 16th IPVS Congress ci-dessus ; incorporés par référence). Parmi les autres valences intéressantes, on peut encore citer Actinobacillus pleuropneumoniae, E. coli et Rhinite atrophique du porc, ou encore maladie d'Aujeszky, peste porcine classique (Hog Choiera), grippe porcine.

La présente invention a aussi pour objet une méthode permettant d'induire une réponse immunitaire chez le porc vis-à-vis des circovirus selon l'invention. Elle a en particulier pour objet une méthode de vaccination efficace chez le porc.

Cette méthode prévoit l'administration au porc, en une ou plusieurs fois, d'un vaccin supra. Il est aussi possible de combiner plusieurs types de vaccins supra dans un même protocole de vaccination.

Cette méthode prévoit non seulement l'administration aux porcs adultes, mais aussi aux jeunes ou aux femelles gestantes, La vaccination de ces dernières permet de conférer une immunité passive aux nouveau-nés (anticorps maternels).

La présente invention offre aussi la possibilité de diagnostiquer la présence des circovirus selon l'invention chez le porc. Elle donne donc accès à des tests de diagnostic et méthodes y relatives mettant en oeuvre les réactifs qui vont être décrits ci-après.

La connaissance des séquences des différents circovirus permet de définir des séquences communes qui permettent de produire des réactifs aptes à reconnaître l'ensemble des circovirus porcins connus.

L'homme du métier pourra aussi choisir des fragments des séquences correspondant à des régions présentant peu ou pas d'homologie avec la séquence correspondante du circovirus PK/15 afin de pouvoir effectuer un diagnostic spécifique.

Les alignements de séquences permettent à l'homme du métier de choisir un réactif conforme à ses souhaits.

Un premier réactif consiste dans les séquences d'ADN divulguées ici et leurs fragments, qui seront notamment utilisés comme sondes ou amorces dans des techniques d'hybridation ou de PCR ("Polymerase Chain Reaction") bien connues.

Un deuxième réactif consiste dans les polypeptides codés par ces séquences à partir du virus ou exprimés à l'aide d'un vecteur (voir supra), ou synthétisés par voie chimique selon les techniques classiques de synthèse peptidique.

Un troisième et quatrième réactifs consistent dans des anticorps respectivement polyclonaux et monoclonaux qui pourront être produits selon les techniques usuelles à partir du virus, des polypeptides ou fragments, extraits ou codés par les séquences d'ADN.

Ces deuxième, troisième et quatrième réactifs pourront être utilisés dans une méthode de diagnostic, dans laquelle l'on recherche, dans un échantillon de fluide physiologique (sang, plasma, sérum, etc.) ou prélèvement de tissu (ganglions, foie, poumons, reins, etc.) provenant d'un porc à tester, la présence d'un antigène spécifique d'un circovirus selon l'invention, en cherchant à détecter soit l'antigène lui-même, soit des anticorps dirigés contre cet antigène.

Les antigènes et anticorps selon l'invention pourront être utilisés dans toutes les techniques de diagnostic de laboratoire connues.

Toutefois, on préférera les mettre à profit dans des techniques pouvant être mises en oeuvre directement sur le terrain par le vétérinaire, l'éleveur ou le propriétaire de l'animal. L'homme du métier dispose de l'ensemble des techniques de laboratoire et du terrain et est donc parfaitement en mesure de les adapter à l'utilisation de cet antigène et/ou des anticorps comme réactif(s) de diagnostic.

Les techniques de diagnostic qui seront préférentiellement utilisées dans le cadre de la présente invention sont le Western Blot, l'immunofluorescence, l'ELISA et l'immunochromatographie.

En ce qui concerne la mise en oeuvre de méthodes par immunochromatographie, le spécialiste pourra se reporter notamment à Robert F. Zurk et al., Clin. Chem. 31/7, 1144-1160 (1985) ainsi qu'aux brevets ou demandes de brevet WO-A-88/08 534, WO-A-91/12528, EP-A-291 176, EP-A-299 428, EP-A-291 194, EP-A-284 232, US-A-5 120 643, US-A-5 030 558, US-A-5 266 497, US-A-4 740 468, US-A-5 266 497, US-A-4 865 240, US-A-5 451 504, US-A-5 141 850, US-A-6 232 835 et US-A-5 238 652.

Ainsi, l'on cherche de préférence à détecter les anticorps spécifiques dans l'échantillon par test indirect, par compétition ou par déplacement. Pour ce faire, on utilise l'antigène lui-même comme réactif de diagnostic, ou un fragment de cet antigène, conservant la reconnaissance des anticorps. Le marquage peut avantageusement être un marquage à la péroxydase ou un marquage particulaire, de préférence à l'or colloïdal.

On peut aussi chercher à détecter l'antigène lui-même dans l'échantillon à l'aide d'un anticorps marqué spécifique de cet antigène. Le marquage est avantageusement comme décrit ci-dessus.

Par anticorps spécifique de l'antigène utilisable notamment en compétition ou déplacement ou pour la détection de l'antigène lui-même, on entend anticorps monoclonaux et polyclonaux spécifiques de l'antigène, fragments de ces anticorps, de préférence fragments Fab ou F(ab)'₂.

Un autre aspect de l'invention est la production d'anticorps, polyclonaux ou monoclonaux, spécifiques de l'antigène conforme à l'invention, ces anticorps pouvant être ensuite utilisés notamment comme réactifs de diagnostic pour la détection de l'antigène dans un échantillon de fluide physiologique ou dans un prélèvement de tissu, ou même pour la détection d'anticorps présents dans un tel échantillon ou prélèvement. L'invention inclut aussi les fragments immunologiquement fonctionnels de ces anticorps, en particulier les fragments F(ab) et F(ab)'₂.

Des anticorps pourront être préparés par les techniques usuelles. On peut notamment se référer à Antibodies, A Laboratory Manual, 1988, Cold Spring Harbor Laboratory, USA ou à J.W. Goding, Monoclonal Antibodies : Principles and Pratice, Academic Press Inc., dont les contenus sont incorporés ici par référence.

On pourra notamment procéder, comme cela est connu en soi, à la fusion de cellules spléniques de souris immunisées par l'antigène ou par au moins l'un de ses fragments, avec des cellules myélomateuses adéquates.

L'invention permet d'obtenir une préparation, de préférence pure ou partiellement purifiée, ou même brute d'anticorps monoclonaux ou polyclonaux spécifiques de l'antigène, notamment anticorps de souris ou de lapin.

La présente invention permet également de déterminer des épitopes d'intérêt notamment sur la base des séquences d'ADN décrites ici, que ce soient des épitopes d'intérêt vaccinal ou des épitopes d'intérêt en diagnostic. A partir de la séquence d'ADN du génome du circovirus selon l'invention, l'homme du métier est à même de déterminer des épitopes selon les méthodes connues par exemple programme informatique approprié ou PEPSCAN. Les épitopes sont des régions immunodominantes de protéines et sont à *ce* titre des régions exposées à la surface des protéines. Ils peuvent être donc reconnus par des anticorps et ainsi être particulièrement employés dans le domaine du diagnostic soit pour la préparation d'anticorps à des fins de diagnostic soit pour la réalisation de peptides correspondants utilisables à titre de réactifs de diagnostic.

Au minimum, un épitope est un peptide ayant de 8 à 9 acides aminés. On préférera en général un minimum de 13 à 25 acides aminés.

L'homme du métier est donc en mesure, en utilisant l'une ou plusieurs de ces techniques ainsi que les autres techniques disponibles, de trouver des épitopes pour la mise en oeuvre de peptides ou d'anticorps à des fins de diagnostic.

L'invention va être maintenant décrite plus en détail à l'aide d'exemples de réalisation non limitatifs, pris en référence au dessin, dans lequel :
**Figure 1** : séquence d'ADN du génome de la souche lmp 1011-48121
**Figure 2** : séquence d'ADN du génome de la souche lmp 1011-48285
**Figure 3** : séquence d'ADN du génome de la souche lmp 999
**Figure 4** : séquence d'ADN du génome de la souche lmp 1010
**Figure 5** : alignement des 4 séquences selon les figures 1 à 4 avec la séquence de la souche PCV PK/15
**Figure 6** : séquence d'ADN du génome de la souche lmp 999 telle que définie dans le premier dépôt en France du 3 octobre 1997
**Figure 7** : Alignements de la séquence de la figure 6 avec la séquence de la souche PK/15

Liste des séquences SEQ ID
**SEQ ID NO: 1** séquence d'ADN du génome de la souche lmp 1011-48121
**SEQ ID NO: 2** séquence d'ADN du génome de la souche lmp 1011-48285
**SEQ ID NO: 3** séquence d'ADN du génome de la souche lmp 999
**SEQ ID NO: 4** séquence d'ADN du génome de la souche lmp 1010
**SEQ ID NO: 5** séquence d'ADN du génome de la souche PK/15
**SEQ ID NO : 6** séquence d'ADN du génome de la souche lmp 999 telle que définie dans le premier dépôt en France du 3 octobre 1997.

### EXEMPLES

### Exemple 1 : Culture et isolement des souches de circovirus porcins:

Des échantillons de tissus ont été récoltés en France, au Canada et aux USA à partir de poumons et de ganglions lymphatiques de porcelets. Ces porcelets présentaient des signes cliniques typiques du syndrome de dépérissement généralisé de post-sevrage. Pour faciliter l'isolement des virus, les échantillons de tissus ont été congelés à -70°C immédiatement après autopsie.

Pour l'isolement viral, des suspensions contenant environ 15% d'échantillon de tissu ont été préparées dans un milieu minimum contenant des sels d'Earl (EMEM, BioWhittaker UK Ltd., Wokingham, UK), de la pénicilline (100 Ul/ml) et de la streptomycine (100 µg/ml)(milieu MEM-SA), par broyage des tissus avec du sable stérile au moyen d'un mortier et d'un pilon stériles. Cette préparation broyée a été alors reprise dans du MEM-SA, puis centrifugée à 3000 g pendant 30 minutes à + 4°C pour récolter le surnageant.

Préalablement à l'ensemencement des cultures de cellules, un volume de 100 µl de chloroforme a été ajouté à 2 ml de chaque surnageant et mélangé en continu pendant 10 minutes à température ambiante. Ce mélange a alors été transféré dans un tube de microcentrifugeuse, centrifugé à 3000 g pendant 10 minutes, puis le surnageant a été récolté. Ce surnageant a été ensuite utilisé comme inoculum pour les expériences d'isolement viral.

Toutes les études d'isolement viral ont été réalisées sur des cultures de cellules PK/15, connues pour être non contaminées par le circovirus porcin (PCV), les pestivirus, les adénovirus porcins et le parvovirus porcin (Allan G. *et al*. Pathogenesis of porcine circovirus expérimental infections of colostrum-deprived piglets and examination of pig foetal material. Vet. Microbiol. 1995. **44**. 49-64).

L'isolement des circovirus porcins a été réalisé selon la technique suivante:

Des monocouches de cellules PK/15 ont été dissociées par trypsination (avec un mélange trypsine-versène) à partir de cultures confluentes, et reprises en milieu MEM-SA contenant 15% de sérum foetal de veau non contaminé par du pestivirus (= milieu MEM-G) sous une concentration finale d'environ 400,000 cellules par ml. Des fractions aliquotes de 10 ml de cette suspension cellulaire ont alors été mélangées avec des fractions aliquotes de 2 ml des inoculums décrits ci-dessus, et les mélanges finaux ont été aliquotes en volumes de 6 ml dans deux flacons Falcon de 25 cm². Ces cultures ont alors été incubées à +37°C pendant 18 heures en atmosphère contenant 10% de CO₂.

Après incubation, le milieu de culture des monocouches semi-confluentes a été traité avec 300 mM de D-glucosamine (Cat # G48175, Sigma-Aldrich Company Limited, Poole, UK) (Tischr I. et al., Arch. Virol. 1987 **96** 39-57), puis l'incubation a été poursuivie pendant une période supplémentaire de 48-72 heures à +37°C. A la suite de cette dernière incubation, l'un des deux Falcons de chaque inoculum a subi 3 cycles successifs de congélation/décongélation. Les cellules PK/15 du Falcon restant ont été traitées avec une solution de trypsine-versène, resuspendues dans 20 ml de milieu MEM-G, puis ensemencées dans des Falcons de 75 cm² à une concentration de 400,000 cellules/ml. Les flacons fraîchement ensemencés ont alors été "surinfectés" par addition de 5 ml du lysat correspondant obtenu après les cycles de congélation/décongélation.

### Exemple 2 : Préparation des échantillons de culture cellulaire pour détection des circovirus porcins par immunofluorescence ou par hybridation in situ.

Un volume de 5 ml de la suspension "surinfectée" a été prélevé et ensemencé dans une boîte de Petri de 55 mm de diamètre contenant une lamelle de verre stérile et dégraissée. Les cultures en flacons et sur lamelles de verre ont été incubées à +37°C et traitées à la glucosamine comme décrit dans l'exemple 1. Les cultures sur lamelles de verre ont été récoltées de 24 à 48 heures après le traitement à la glucosamine et fixées, soit avec de l'acétone pendant 10 minutes à température ambiante, soit avec 10% de formaldéhyde tamponné pendant 4 heures. Suite à cette fixation, toutes les lamelles de verre ont été stockées à -70°C, sur gel de silice, avant leur utilisation pour les études d'hybridation *in situ* et les études de marquage immunocytochimique.

### Exemple 3 : Techniques de détection de séquences PCV par hybridation in situ

L'hybridation *in situ* a été réalisée sur les tissus prélevés sur les porcs malades et fixés au formaldéhyde et également sur les préparations de cultures de cellules inoculées pour l'isolement viral (voir exemple 2) et fixées sur lamelles de verre.

Des sondes génomiques complètes correspondant aux circovirus porcin PK/15 (PCV) et au virus de l'anémie infectieuse du poulet (chicken anemia virus = CAV) ont été utilisées. Le plasmide pPCV1, contenant la forme réplicative du génome PCV clonée sous la forme d'un insert unique de 1,7 kilopaires de bases (kpb) (Meehan B. *et al*. Sequence of porcine circovirus DNA: affinities with plant circoviruses. J. Gen. Virol. 1997. **78**. 221-227) a été utilisé comme source d'ADN viral spécifique pour PCV. Un plasmide analogue, pCAA1, contenant la forme réplicative 2,3 kpb du circovirus aviaire CAV a été utilisé comme contrôle négatif. Les stocks de glycérols respectifs de ces deux plasmides ont été utilisés pour la production et la purification des plasmides selon la technique de lyse alcaline (Sambrook J. *et al*. Molecular cloning : A Laboratory Manual. 2^{nd} Edition. Cold Spring Harbor Laboratory. Cold Spring Harbor. New york. 1989) afin qu'ils servent ensuite de matrices pour la préparation des sondes. Les sondes circovirus représentatives des génomes complets du PCV et de CAV ont été produites à partir des plasmides purifiés décrits ci-dessus (1 µg pour chaque sonde) et d'amorces hexanucléotidiques au hasard en utilisant un kit commercial de marquage non radioactif ("DIG DNA labelling kit" , Boehringer Mannheim, Lewes, UK) selon les recommandations du fournisseur.

Les sondes marquées à la digoxigénine ont été reprises sous un volume de 50-100 µl d'eau stérile avant leur utilisation pour l'hybridation *in situ*.

Les échantillons de tissus de porcs malades, inclus dans la paraffine et fixés au formaldéhyde, ainsi que les préparations de cultures de cellules infectées, fixées au formaldéhyde, ont été préparées pour la détection des acides nucléiques PCV selon la technique suivante :

Des sections de 5 µm d'épaisseur ont été découpées à partir des blocs de tissus inclus dans la paraffine, déparaffinés, puis réhydratés dans des solutions successives d'alcool à concentration décroissante. Les sections de tissus et les cultures de cellules fixées au formaldéhyde ont été incubées respectivement pendant 15 minutes et 5 minutes à +37°C dans une solution de protéinase K à 0,5% en tampon Tris-HCl 0,05M, EDTA 5 mM (pH 7,6). Les lames ont été alors placées dans une solution de glycine à 1% en eau distillée autoclavée, pendant 30 secondes, lavées deux fois avec un tampon PBS (phosphate buffer saline) 0,01 M (pH 7,2), et enfin lavées pendant 5 minutes en eau distillée stérile. Elles ont été finalement séchées à l'air libre et mises en contact avec les sondes.

Chaque préparation tissu/sonde a été recouverte avec une lamelle propre et dégraissée, puis placée dans un four à +90°C pendant 10 minutes, mise ensuite en contact avec un bloc de glace pendant 1 minute, et enfin incubée pendant 18 heures à +37°C. Les préparations ont été ensuite immergées brièvement dans un tampon sel de sodium-citrate (SSC) 2X (pH 7,0) pour éliminer les lamelles protectrices, puis lavées 2 fois pendant 5 minutes en tampon SSC 2X et enfin lavées 2 fois pendant 5 minutes en tampon PBS.

Après ces lavages, les préparations ont été immergées dans une solution d'acide maléique 0,1 M, NaCl 0,15 M (pH 7,5) (tampon maléique) pendant 10 minutes, puis incubées dans une solution de 1% de réactif bloquant (Cat # 1096176, Boehringer Mannheim UK, Lewis, East Sussex, UK) en tampon maléique pendant 20 minutes à +37°C.

Les préparations ont alors été incubées avec une solution au 1/250 d'un anticorps monoclonal anti-digoxigénine (Boehringer Mannheim), dilué en tampon bloquant, pendant 1 heure à +37°C, lavées en PBS et enfin incubées avec un anticorps biotinylé anti-immunoglobuline de souris pendant 30 minutes à + 37°C. Les préparations ont été lavées en PBS et l'activité péroxydase endogène a été bloquée par un traitement avec une solution de péroxyde d'hydrogène à 0,5% en PBS pendant 20 minutes à température ambiante. Les préparations ont été lavées une nouvelle fois en PBS et traitées avec un substrat 3-amino-9-diéthylcarbazole (AEC) (Cambridge Bioscience, Cambridge, UK) préparé extemporanément.

Après un dernier lavage à l'eau de ville, les préparations ont été contre-colorées avec de l'hématoxyline, "bleuies" sous eau de ville, et montées sous lamelles microscopiques avec un liquide de montage (GVA Mount, Cambridge Bioscience, Cambridge, UK). Les contrôles d'expérience ont inclus l'utilisation d'une sonde négative non pertinente (CAV) et d'une sonde positive (PCV) sur des échantillons provenant de porcs malades et de porcs non malades.

### Exemple 4 : Technique de détection du PCV par immunofluorescence

Le criblage initial de toutes les préparations de culture cellulaire fixées à l'acétone a été réalisé par une technique d'immunofluorescence indirecte (IFI) utilisant une dilution au 1/100 d'un pool de sérums de porcs adultes. Ce pool de sérums comprend des sérums de 25 truies adultes d'Irlande du Nord et est connu pour contenir des anticorps contre une grande variété de virus porcins, y compris PCV : parvovirus porcin, adénovirus porcin, et virus PRRS. La technique IFI a été réalisée par un contact du sérum (dilué en PBS) avec les cultures cellulaires pendant une heure à +37°C, suivi de deux lavages en PBS. Les cultures de cellules sont alors colorées avec une dilution au 1/80 en PBS d'un anticorps de lapin anti-immunoglobuline de porc conjugué à de l'isothiocyanate de fluorescéine pendant une heure, puis lavées en PBS et montées en tampon glycérol préalablement à l'observation microscopique sous éclairage ultra-violet.

### Exemple 5 : Résultats de l'hybridation in situ sur les tissus de porcs malades

L'hybridation *in situ,* utilisant une sonde génomique PCV, réalisée sur des tissus prélevés sur des porcelets français, canadiens et californiens présentant des lésions de dépérissement généralisé et fixés au formaldéhyde, a révélé la présence d'acides nucléiques PCV associés aux lésions, dans plusieurs des lésions étudiées. Aucun signal n'a été observé lorsque la sonde génomique PCV a été utilisée sur des tissus prélevés sur des porcs non malades ou lorsque la sonde CAV a été utilisée sur les tissus de porcs malades. La présence d'acide nucléique PCV a été identifiée dans le cytoplasme et le noyau de nombreuses cellules mononucléaires infiltrant les lésions dans les poumons des porcelets californiens. La présence d'acide nucléique PCV a également été mise en évidence dans les pneumocytes, les cellules épithéliales bronchiques et bronchiolaires, et dans les cellules endothéliales des petites artérioles, veinules et vaisseaux lymphatiques.

Chez les porcs malades français, la présence d'acide nucléique PCV a été détectée dans le cytoplasme de nombreux lymphocytes folliculaires et dans les cellules mononucléaires intrasinusoïdales des ganglions lymphatiques. L'acide nucléique PCV a également été détecté dans des syncytia occasionnels. En fonction de ces résultats de détection, des échantillons de poumons de porcs californiens, de ganglions lymphatiques mésentériques de porcs français, et d'organes de porcs canadiens ont été choisis aux fins d'isolement des nouvelles souches de circovirus porcin.

### Exemple 6 : Résultats de la culture cellulaire des nouvelles souches de circovirus porcin et détection par immunofluorescence

Aucun effet cytopathique (ECP) n'a été observé dans les cultures de cellules inoculées avec les échantillons prélevés sur les porcelets francais (souche lmp.1008), californiens (souche lmp.999) et canadiens (souche lmp.1010) montrant des signes cliniques du syndrome du dépérissement généralisé. Cependant, l'immuno-marquage des préparations provenant des cultures de cellules inoculées, après fixation à l'acétone et avec un pool de sérums polyclonaux de porcs, a révélé une fluorescence nucléaire chez de nombreuses cellules dans les cultures inoculées à partir des poumons de porcelets californiens (souche Imp.999), à partir des ganglions lymphatiques médiastinaux des porcelets français (souche Imp. 1008), et à partir d'organes des porcelets canadiens (souche lmp.1010).

### Exemple 7 : Extraction de l'ADN génomique des circovirus porcins

Les formes réplicatives des nouvelles souches de circovirus porcin (PCV) ont été préparées à partir de cultures de cellules PK/15 infectées (voir exemple 1) (10 Falcons de 75 cm²) récoltées après 72-76 heures d'incubation et traitées à la glucosamine, comme décrit pour le clonage de la forme réplicative du CAV (Todd. D. *et al*. Dot blot hybridization assay for chicken anemia agent using a cloned DNA probe. J. Clin. Microbiol. 1991. **29**. 933-939). L'ADN double brin de ces formes réplicatives a été extrait selon une modification de la technique de Hirt (Hirt B. Sélective extraction of polyoma virus DNA from infected cell cultures. J. Mol. Biol. 1967. **36**. 365-369), comme décrit par Molitor (Molitor T.W. *et al*. Porcine parvovirus DNA: characterization of the genomic and replicative form DNA of two virus isolates. Virology. 1984. **137**. 241-254).

### Exemple 8 : Carte de restriction de la forme réplicative du génome de la souche Imp.999 de circovirus porcin.

L'ADN (1-5 µg) extrait selon la technique de Hirt a été traité par la nucléase S1 (Amersham) selon les recommandations du fournisseur, puis cet ADN a été digéré par différentes enzymes de restriction (Boehringer Mannheim, Lewis, East Sussex, UK) et les produits de la digestion ont été séparés par électrophorèse sur gel d'agarose à 1,5% en présence de bromure d'éthidium comme décrit par Todd *et al*. (Purification and biochemical characterization of chicken anémia agent. J. Gen. Virol. 1990. **71**. 819-823).
L'ADN extrait des cultures de la souche lmp.999 possède un site unique EcoRI, 2 sites Sacl et ne possède pas de site Pstl. Ce profil de restriction est donc différent du profil de restriction présenté par la souche PCV PK/15 (Meehan B. *et al*. Sequence of porcine circovirus DNA: affinities with plant circoviruses. 1997. **78**. 221-227) qui possède au contraire un site Pstl et ne possède pas de site EcoRI.

### Exemple 9 : Clonage du génome de la souche Imp.999 de circovirus porcin

Le fragment de restriction d'environ 1,8 kpb généré par digestion de la forme réplicative double brin de la souche PCV Imp.999 avec l'enzyme de restriction EcoRI a été isolé après électrophorèse sur gel d'agarose à 1,5% (voir exemple 3) en utilisant un kit commercial Qiagen (QIAEXII Gel Extraction Kit, Cat # 20021,QIAGEN Ltd., Crawley, West Sussex, UK). Ce fragment de restriction EcoRI-EcoRI a été ensuite ligaturé avec le vecteur pGEM-7 (Promega, Medical Supply Company, Dublin, Ireland), préalablement digéré par les mêmes enzymes de restriction et déphosphorylé, en suivant les techniques standards de clonage (Sambrook J. *et al*. Molecular cloning : A Laboratory Manual. 2^{nd} Edition. Cold Spring Harbor Laboratory. Cold Spring Harbor. New york. 1989). Les plasmides obtenus ont été transformés dans une souche hôte *Escherichia coli* JM109 (Stratagene, La Jolla, USA) selon les techniques standards. Le fragment de restriction EcoRI-EcoRI de la souche PCV lmp.999 a également été cloné dans le site EcoRI du vecteur pBlueScript SK+ (Stratagene inc. La Jolla, USA). Parmi les clones obtenus pour chaque souche hôte, au moins 2 clones contenant les fragments de la taille attendue ont été sélectionnés. Les clones obtenus ont alors été cultivés et les plasmides contenant le génome complet de la souche lmp.999 ont été purifiés en petit volume (2 ml) ou en grand volume (250 ml) selon les techniques standards de préparation et de purification des plasmides.

### Exemple 10 : Séquençage de l'ADN génomique (forme réplicative double brin) de la souche PCV Imp.999.

La séquence nucléotidique de 2 clones EcoRI lmp.999 (clones pGEM-7/2 et pGEM-7/8) a été déterminée selon la technique des didéoxynucléotides de Sanger en utilisant le kit de séquençage "AmpliTaq DNA polymerase FS" (Cat # 402079 PE Applied Biosystems, Warrington, UK) et un appareil de séquençage automatique Applied BioSystems ABI373A selon les recommandations du fournisseur. Les réactions de séquençage initiales ont été faites avec les primers universels M13 "forward" et "reverse". Les réactions de séquençage suivantes ont été générées selon la technique de "marche sur l'ADN". Les oligonucléotides nécessaires à ces séquençages ultérieurs ont été synthétisés par Life Technologies (Inchinnan Business Park, Paisley, UK).

Les séquences générées ont été assemblées et analysées au moyen du logiciel MacDNASIS version 3.2. (Cat # 22020101, Appligene, Durham, UK), Les différents cadres ouverts de lecture ont été analysés au moyen de l'algorithme BLAST disponible sur le serveur du "National Center for Biotechnology Information (NCBI, Bethesda, MD, USA).

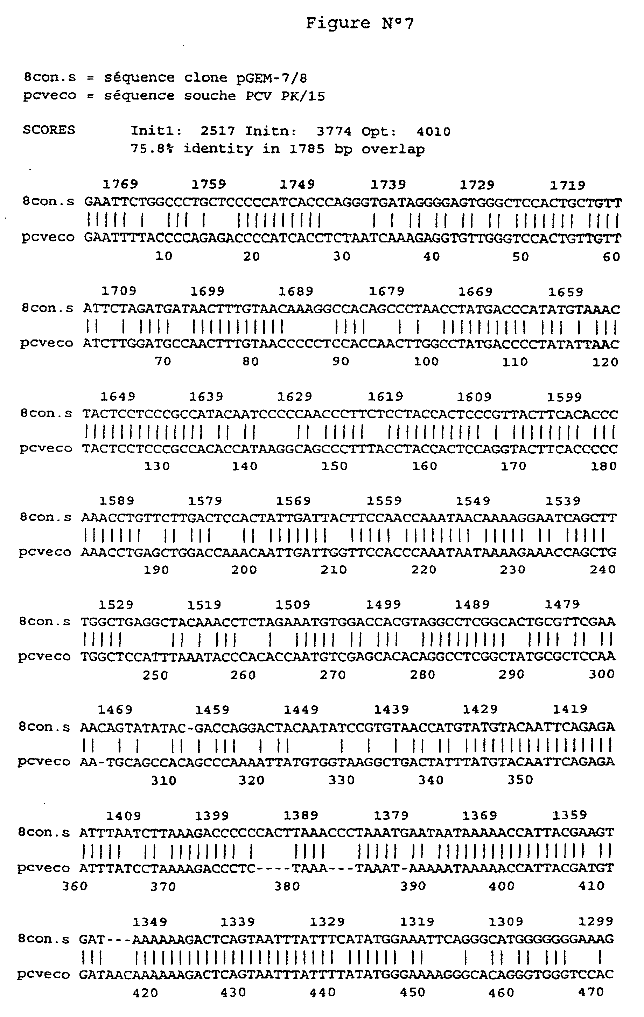

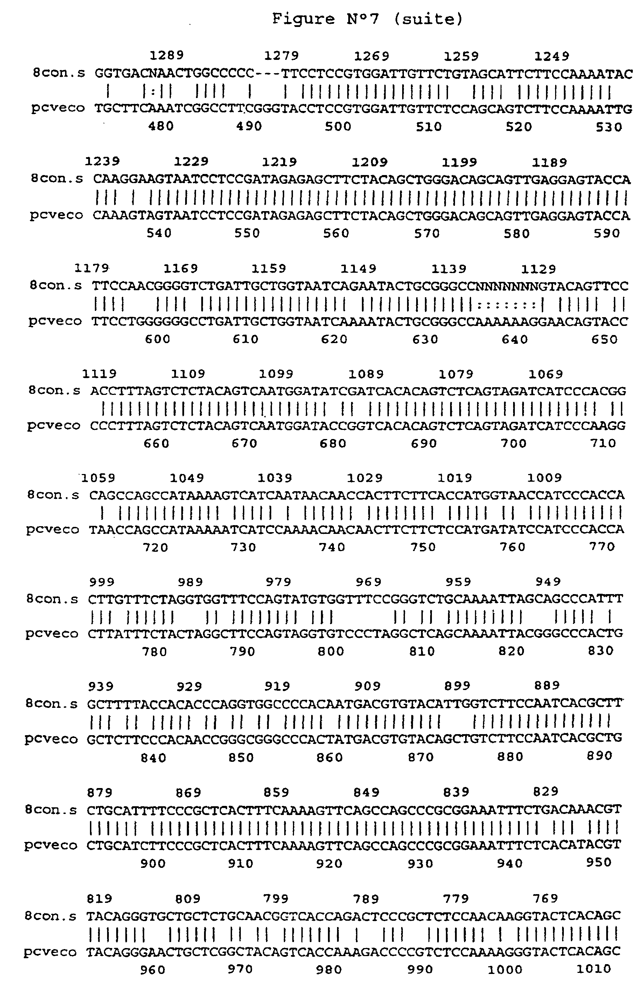

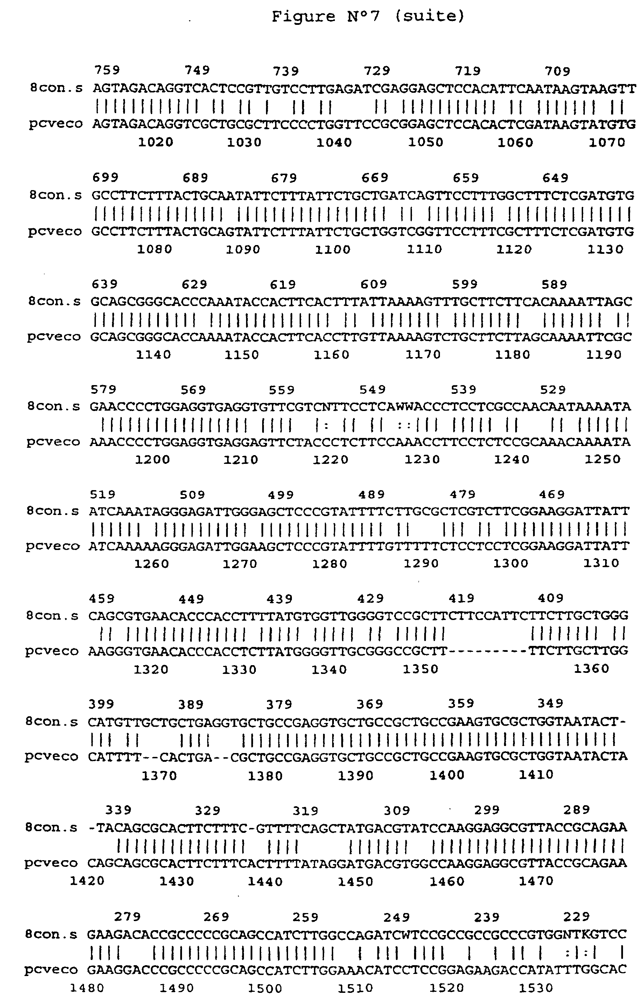

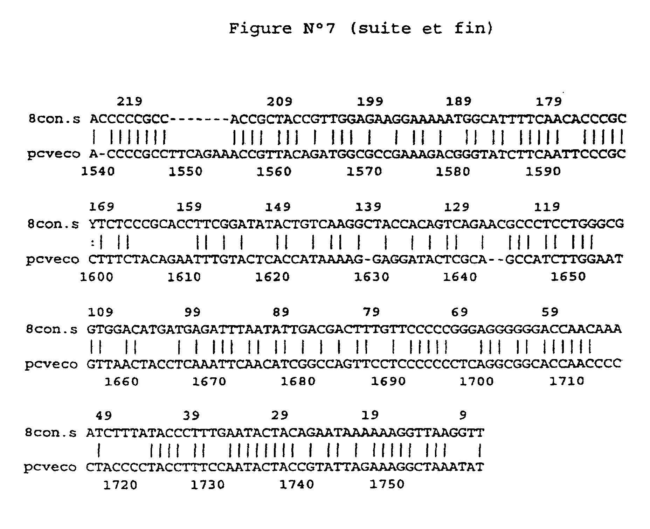

La séquence complète (fragment EcoRI-EcoRt) obtenue initialement à partir du clone pGEM-7/8 (SEQ ID NO : 6) est présentée sur la figure N°6. Elle débute arbitrairement après le G du site EcoRI et présente quelques incertitudes sur le plan des nucléotidiques.

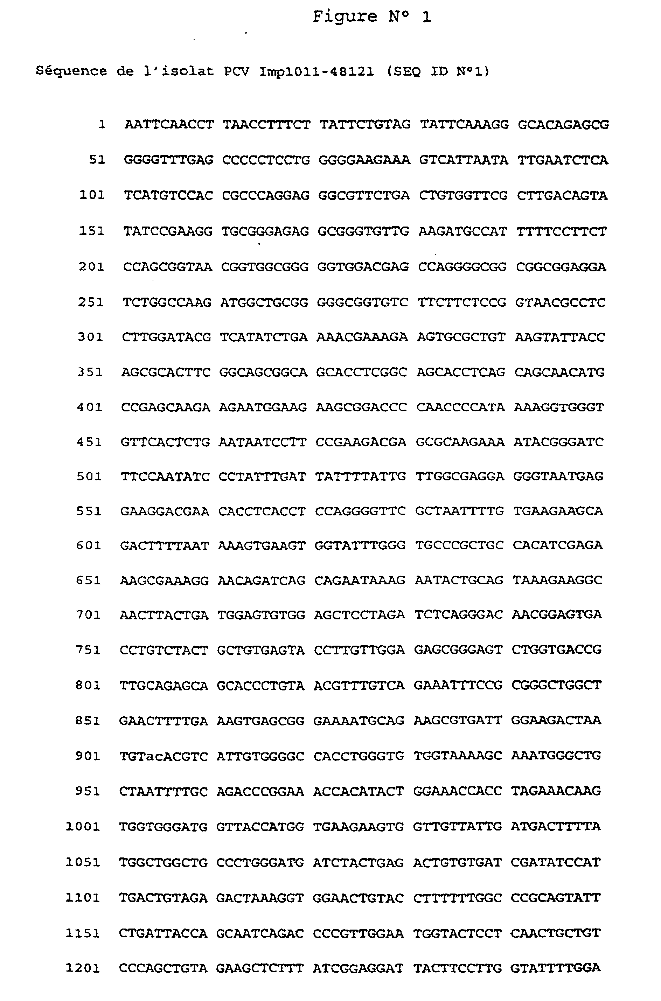

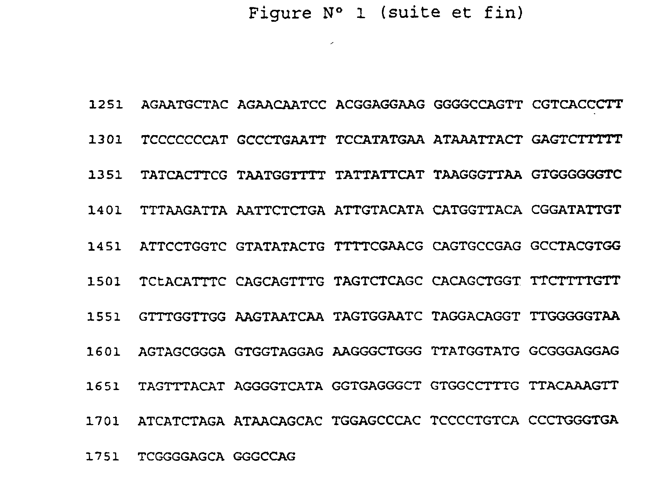

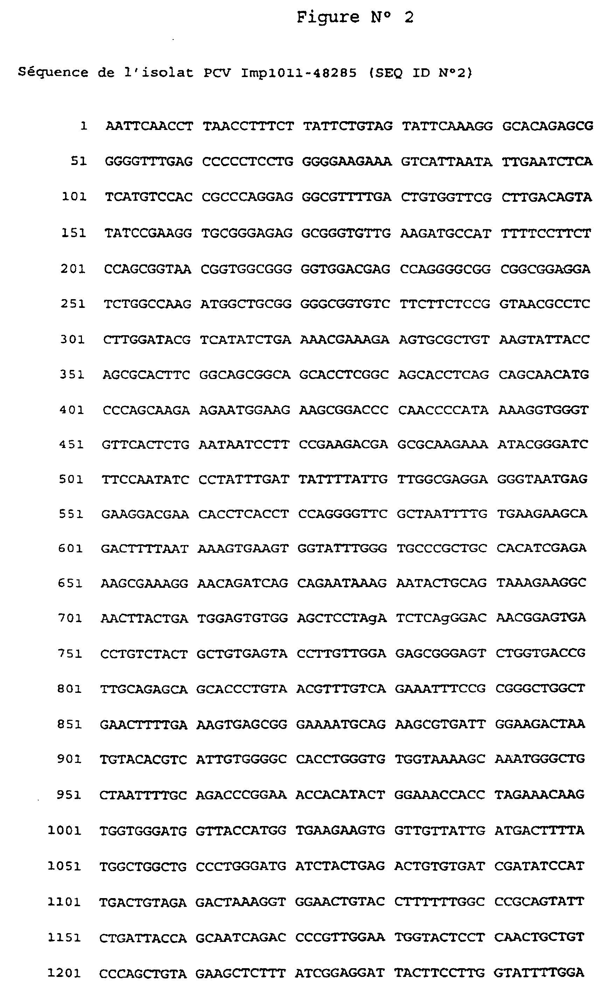

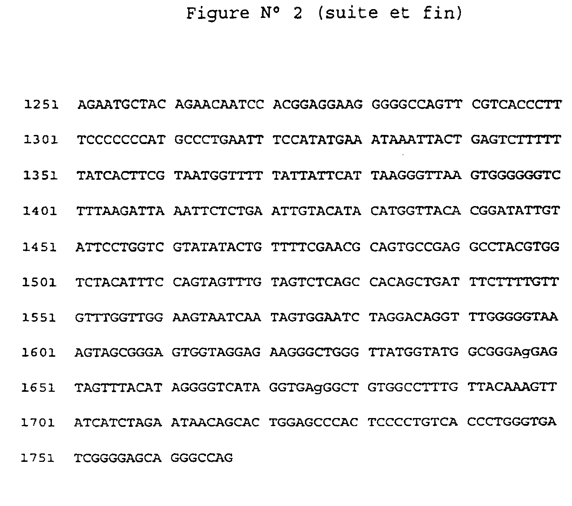

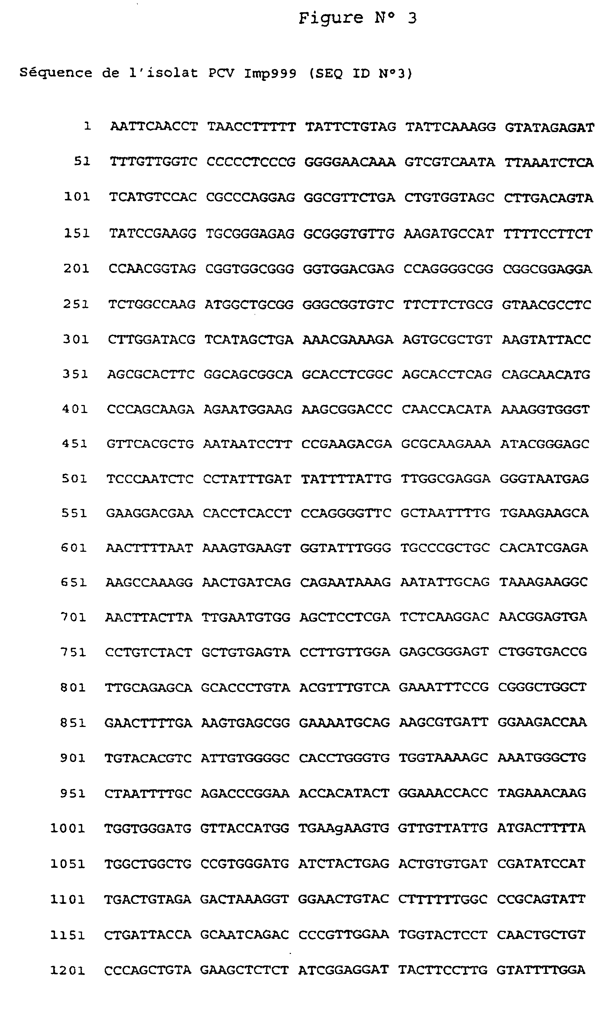

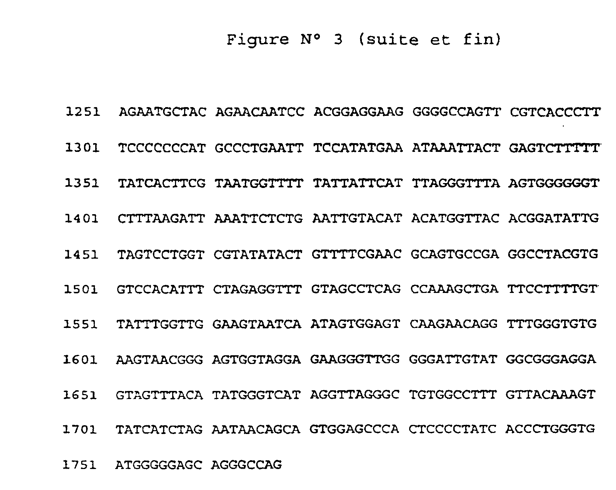

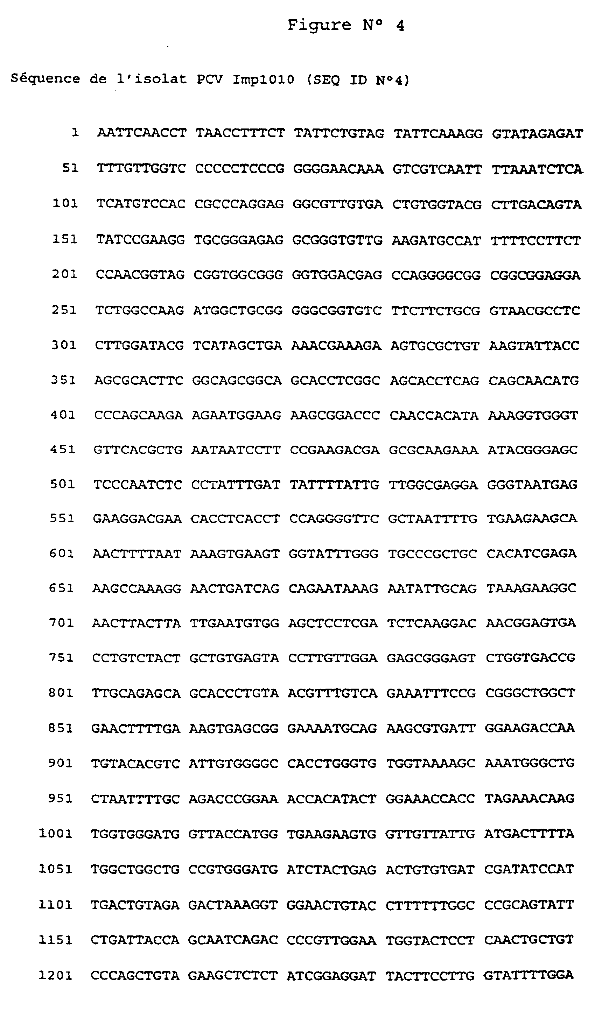

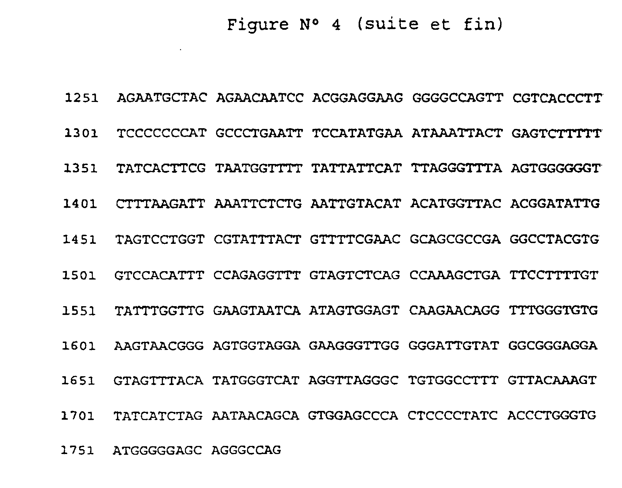

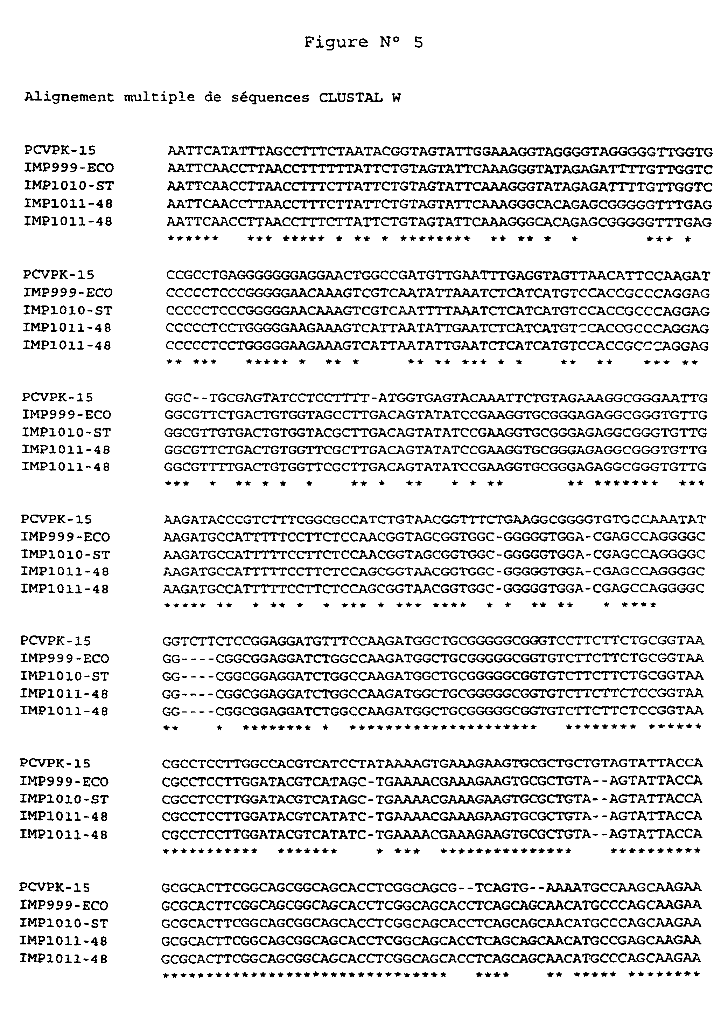

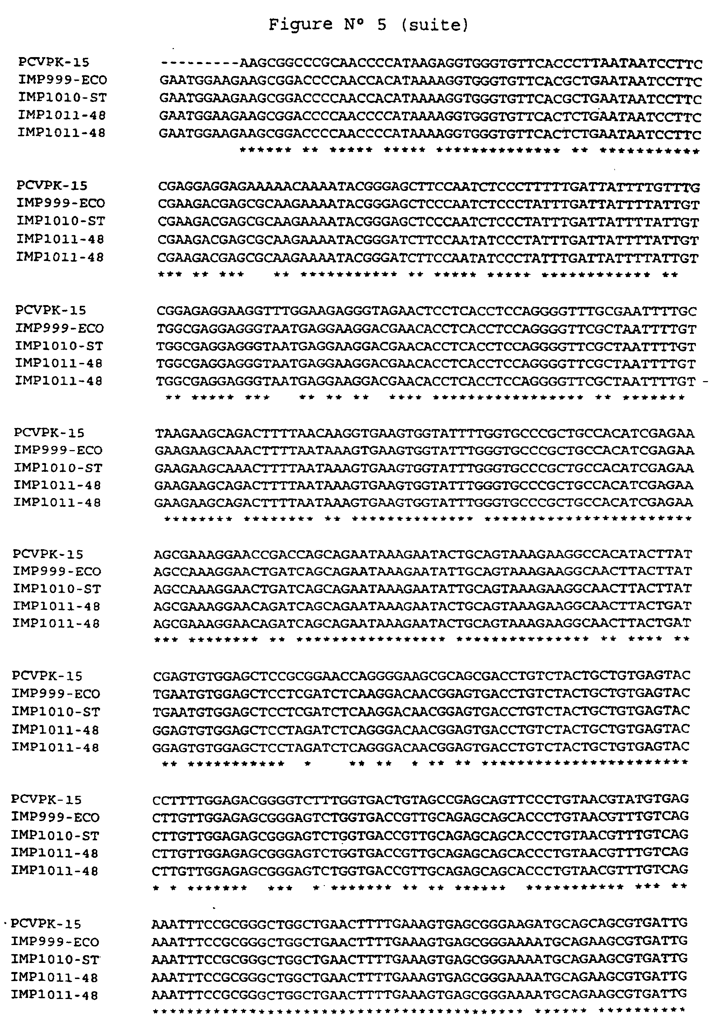

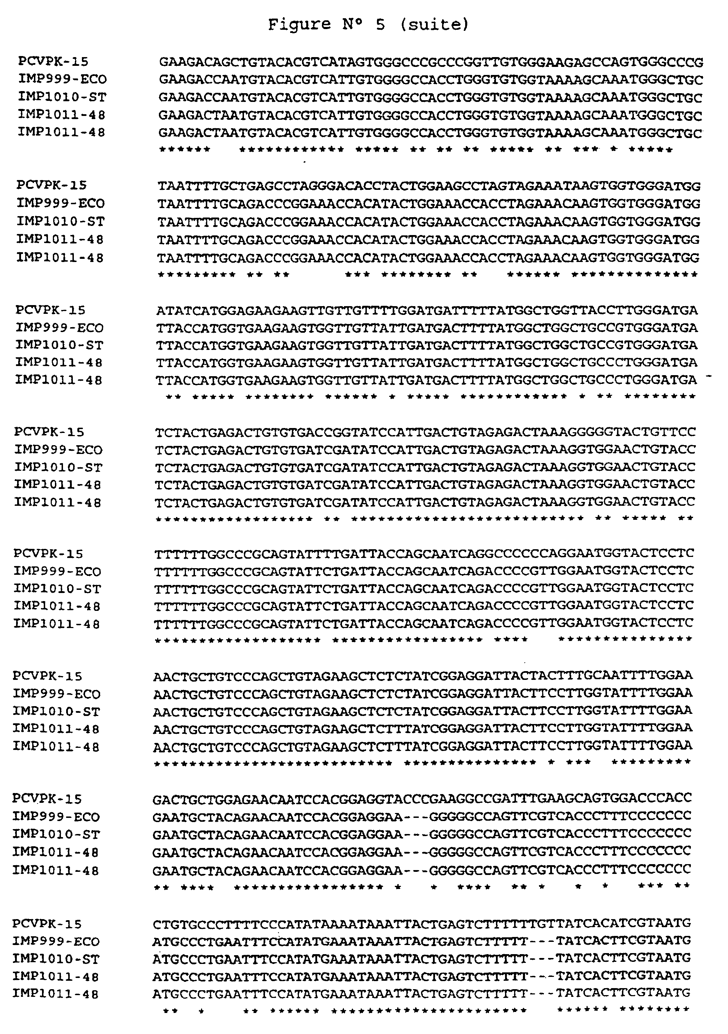

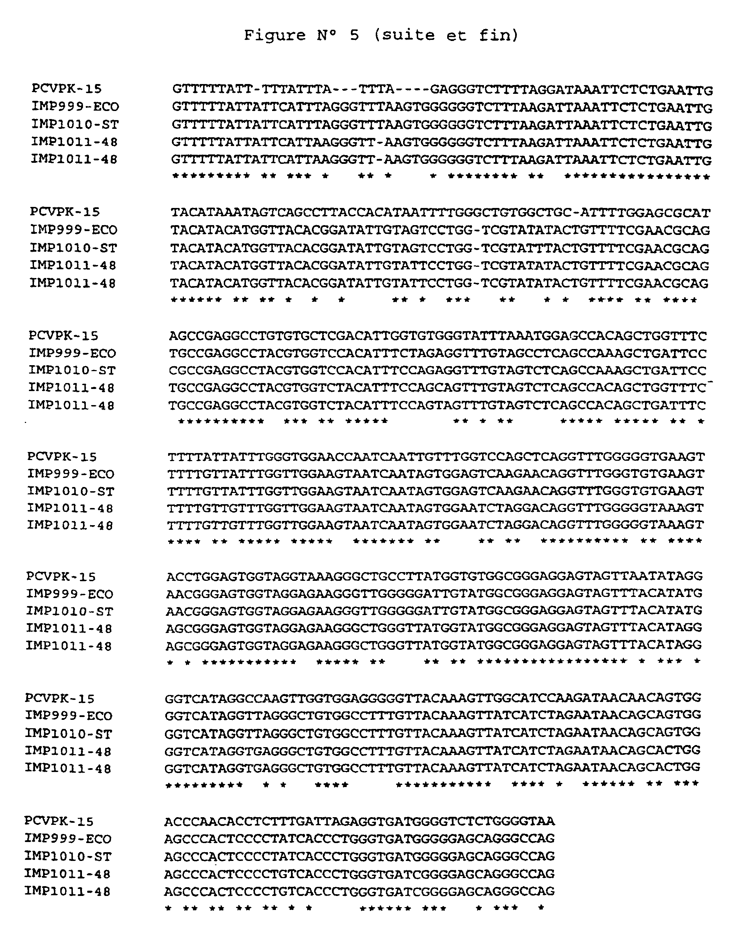

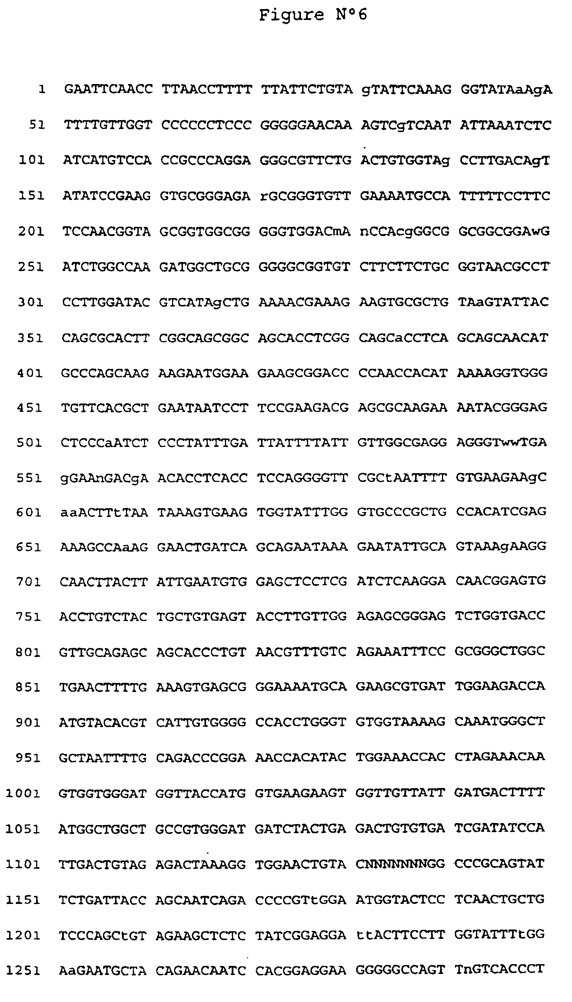

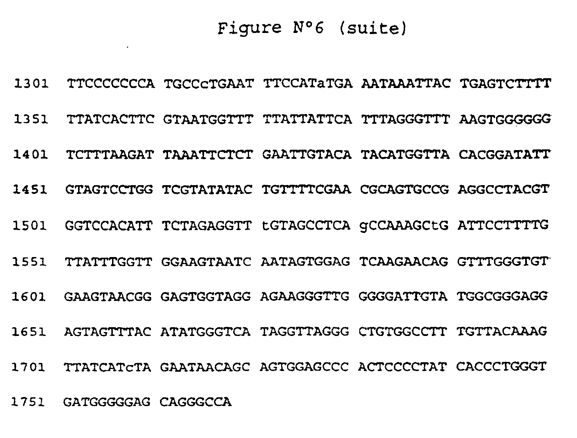

Le séquençage a ensuite été optimisé et la SEQ ID NO : 3 (Figure 3) donne la séquence totale de cette souche, que l'on a fait débuté arbitrairement au début du site EcoRI, soit le G comme premier nucléotide.

On a procédé d'une manière similaire pour l'obtention de la séquence des trois autres isolats selon l'invention (voir SEQ ID NO : 1, 2 et 4 et figures 1, 2 et 4).

La taille du génome de ces quatre souches est :
lmp 1011-48121 1767 nucléotides
lmp 1011-48285 1767 nucléotides
lmp 999 1768 nucléotides
lmp 1010 1768 nucléotides

### Exemple 11 : Analyse de la séquence de la souche PCV Imp.999.

Lorsque la séquence générée à partir de la souche lmp.999 a été utilisée pour une recherche d'homotogie vis-à-vis des séquences contenues dans la banque de données GenBank, la seule homologie significative qui ait été détectée est une homologie d'environ 76 % (au niveau acide nucléique) avec la séquence de la souche PK/15 (Numéros d'accès Y09921 et U49186) (voir figure N°5).

Au niveau acides aminés, la recherche d'homologie de la traduction des séquences dans les 6 phases avec les banques de données (algorithme BLAST X sur le serveur NCBI) a permis de mettre en évidence une homologie de 94 % avec le cadre ouvert de lecture correspondant à la réplicase théorique du virus BBTV similaire aux circovirus de plantes (numéro d'identification GenBank 1841515) codée par la séquence GenBank U49186.

Aucune autre séquence contenue dans les banques de données ne montre d'homologie significative avec la séquence générée à partir de la souche PCV Imp.999.

L'analyse des séquences obtenues à partir de la souche lmp.999 cultivée à partir de lésions prélevées sur des porcelets californiens présentant des signes cliniques du syndrome de dépérissement généralisé montre clairement que cet isolat viral est une nouvelle souche de circovirus porcin.

### Exemple 12 : Analyse comparative des séquences

L'alignement des séquences nucléotidiques des 4 nouvelles souches PCV a été fait avec la séquence de la souche PCV PK/15 (figure 5). Une matrice d'homologie prenant en compte les quatre nouvelles souches et la souche antérieure PK/15 a été réalisée. Les résultats sont les suivants :
1 : lmp 1011-48121
2 : lmp 1011-48285
3 : lmp 999
4 : lmp 1010
5 : PK/15

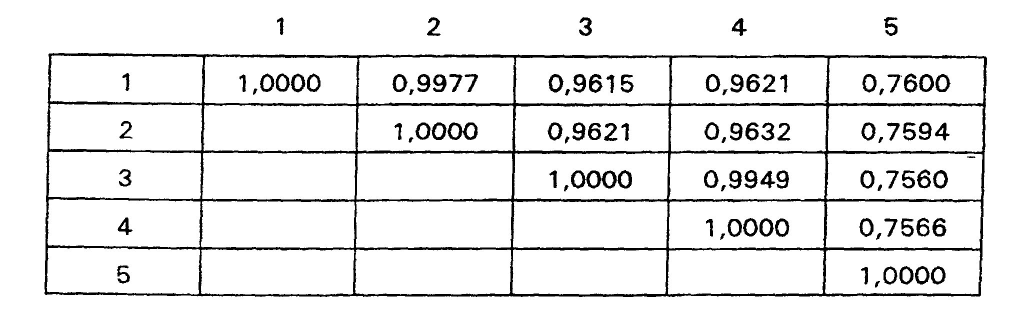

L'homologie entre les deux souches françaises lmp 1011-48121 et lmp 1011-48285 est supérieure à 99 % (0,9977).

L'homologie entre les deux souches nord-américaines lmp 999 et lmp 1010 est aussi supérieure à 99 % (0,9949). L'homologie entre souches françaises et souches nord-américaines est un peu supérieure à 96 %.

L'homologie de toutes ces souches avec PK/15 tombe à une valeur comprise entre 75 et 76 %.

On en déduit que les souches selon l'invention sont représentatives d'un nouveau type de circovirus porcin, distinct du type représenté par la souche PK/15. Ce nouveau type, isolé de porcs présentant le syndrome PMWS, est dénommé circovirus porcin de type II, la PK/15 représentant le type I. Les souches appartenant à ce type II présentent une remarquable homogénéité de séquence nucléotidique, alors même qu'elles ont été isolées dans des régions géographiques très éloignées.

### Exemple 13 : Analyse des protéines codées par le génome des nouvelles souches PCV.

La séquence nucléotidique de l'isolat lmp. 1010 a été considérée comme représentative des autres souches de circovirus associées au syndrome de dépérissement généralisé. Cette séquence a été analysée plus en détail à l'aide de l'algorithme BLASTX (Altschul *et al*. J. Mol. Biol. 1990. **215**. 403-410) et d'une combinaison de programmes de l'ensemble de logiciels MacVector 6.0 (Oxford Molecular Group, Oxford OX4 4GA, UK). Il a été possible de détecter 13 cadres ouverts de lecture (ou COLs) d'une taille supérieure à 20 acides aminés sur cette séquence (génome circulaire). Ces 13 COLs sont les suivants :

| Nom | Début | Fin | Brin | Taille du COL (nucléotides (nt)) | Taille protéine (acides aminés (aa)) |
|---|---|---|---|---|---|
| COL1 | 103 | 210 | sens | 108 nt | 35 aa |
| COL2 | 1180 | 1317 | sens | 138 nt | 45 aa |
| COL3 | 1363 | 1524 | sens | 162 nt | 53 aa |
| COL4 | 398 | 1342 | sens | 945 nt | 314 aa |
| COL5 | 900 | 1079 | sens | 180 nt | 59 aa |
| COL6 | 1254 | 1334 | sens | 81 nt | 26 aa |
| COL7 | 1018 | 704 | antisens | 315 nt | 104 aa |
| COL8 | 439 | 311 | antisens | 129 nt | 42 aa |
| COL9 | 190 | 101 | antisens | 90 nt | 29 aa |
| COL10 | 912 | 733 | antisens | 180 nt | 59 aa |
| COL11 | 645 | 565 | antisens | 81 nt | 26 aa |
| COL12 | 1100 | 1035 | antisens | 66 nt | 21 aa |
| COL13 | 314 | 1381 | antisens | 702 nt | 213 aa |

Les positions de début et de fin de chaque COL se réfèrent à la séquence présentée sur la figure N° 4 (SEQ ID N° 4), du génome de la souche 1010. Les limites des COLs 1 à 13 sont identiques pour la souche 999. Elles le sont aussi pour les souches 1011-48121 et 1011-48285, sauf pour les COLs 3 et 13 :
COL3 1432-1539, sens, 108 nt, 35aa
COL13 314-1377, antisens, 705 nt, 234 aa.

Parmi ces 13 COLs, 4 présentent une homologie significative avec des COLs analogues situés sur le génome du virus cloné PCV PK-15. Chacun des cadres ouverts de lecture présents sur le génome de tous les isolats de circovirus associés au syndrome de dépérissement généralisé a été analysé. Ces 4 COLs sont les suivants :

| Nom | Début | Fin | Brin | Taille du COL (nt) | Taille protéine (acides aminés) | Masse moléculaire |
|---|---|---|---|---|---|---|
| COL4 | 398 | 1342 | sens | 945 nt | 314 aa | 37,7 kDa |
| COL7 | 1018 | 704 | antisens | 315 nt | 104 aa | 11,8 kDa |
| COL10 | 912 | 733 | antisens | 180 nt | 59 aa | 6,5 kDa |
| COL13 | 314 | 1381 | antisens | 702 nt | 233 aa | 27,8 kDa |

Les positions de début et de fin de chaque COL se réfèrent à la séquence présentée sur la figure N° 4 (SEQ ID N ° 4). La taille du COL (en nucléotides = nt) inclut le codon stop.

La comparaison entre l'organisation génomique des isolats PCV lmp. 1010 et PCV PK-15 a permis l'identification de 4 COLs conservés dans le génome des deux virus. Le tableau ci-dessous présente les degrés d'homologie observés:

| **COL Imp.1010/COL PCV PK-15** | **Pourcentage d'homologie** |
|---|---|
| COL4/COL1 | 86 % |
| COL13/COL2 | 66,4% |
| COL7/COL3 | 61,5 % (au niveau du recouvrement (104 aa) |
| COL10/COL4 | 83 % (au niveau du recouvrement (59 aa) |

La plus grande identité de séquence a été observée entre le COL4 Imp. 1010 et le COL1 PK-15 (86% d'homologie). Ceci était attendu dans la mesure où cette protéine est probablement impliquée dans la réplication de l'ADN viral et est essentielle pour la réplication virale (Meehan *et al*. J. Gen. Virol. 1997. **78**. 221-227; Mankertz *et al*. J. Gen. Virol. 1998. **79**. 381-384).

L'identité de séquence entre le COL13 lmp. 1010 et le COL2 PK-15 est moins forte (66,4% d'homologie), mais chacun de ces deux COLS présente bien une région basique N-terminale très conservée, qui est identique à la région N-terminale de la protéine structurale majeure du circovirus aviaire CAV (Meehan *et al*. Arch. Virol. 1992. **124**. 301-319). De plus grandes différences sont observées entre COL7 lmp. 1010 et COL3 PK-15 et entre COL10 Imp. 1010 et COL4 PK-15. Dans chaque cas, il existe une délétion de la région C-terminale des COL7 et COL10 de l'isolat Imp. 1010 lorsqu'on les compare aux COL3 et COL4 de PCV PK-15. La plus haute homologie de séquence est observée au niveau des régions N-terminales de COL7/COL3 (61,5% d'homologie au niveau du recouvrement) et de COL10/COL4 (83% d'homologie au niveau du recouvrement).

Il apparaît que l'organisation génomique du circovirus porcin est assez complexe suite à l'extrême compacité de son génome. La protéine structurale majeure est probablement issue d'un épissage entre plusieurs cadres de lecture situés sur le même brin du génome du circovirus porcin. On peut donc considérer que tout cadre ouvert de lecture (COL1 à COL13) tel que décrit dans le tableau ci-dessus, peut représenter tout ou partie d'une protéine antigénique codée par le circovirus porcin de type II et est donc potentiellement un antigène utilisable pour le diagnostic spécifique et/ou pour la vaccination. L'invention concerne donc toute protéine comprenant au moins un de ces COLs. De préférence, l'invention concerne une protéine formée essentiellement par les COL4, COL7, COL10 ou COL13.

### Exemple 14 : Caractère infectieux du génome PCV cloné à partir des nouvelles souches.

Le plasmide pGEM-7/8 contenant le génome complet (forme réplicative) de l'isolat Imp.999 a été transfecté dans des cellules PK/15 selon la technique décrite par Meehan B. *et al*. (Characterization of viral DNAs from cells infected with chicken anemia agent : sequence analysis of the cloned replicative form and transfection capabilities of cloned genome fragments. Arch. Virol. 1992. **124.** 301-319). L'analyse par immunofluorescence (voir exemple 4) réalisée sur le premier passage après transfection sur cellules PK/15 non contaminées a montré que le plasmide du clone pGEM7/8 était capable d'induire la production de virus PCV infectieux. La disponibilité d'un clone contenant un matériel génétique PCV infectieux permet toute manipulation utile sur le génome viral afin de produire des virus PCV modifiés (soit atténués chez le porc, soit défectifs) utilisables pour la production de vaccins atténués ou recombinés, ou pour la production d'antigènes pour des trousses de diagnostic.

### Exemple 15 : Production des antigènes PCV par culture in vitro

La culture des cellules PK/15 non contaminées et la multiplication virale sont réalisées selon les mêmes modalités qu'à l'exemple 1. Les cellules infectées sont récoltées après trypsination après 4 jours d'incubation à 37 °C et numérées. Le passage suivant est inoculé avec 400 000 cellules infectées par ml.

### Exemple 16 : Inactivation des antigènes viraux

En fin de culture virale, les cellules infectées sont récoltées et lysées par ultrasons (Branson Sonifier) ou à l'aide d'un broyeur colloïdal de type rotor-stator (UltraTurrax, IKA). La suspension est ensuite centrifugée à 3700 g pendant 30 minutes. La suspension virale est inactivée par 0,1 % d'éthylène imine pendant 18 heures à +37°C ou par 0,5 % de bêta-propiolactone pendant 24 heures à +28°C. Si le titre du virus avant inactivation est insuffisant, la suspension virale est concentrée par ultrafiltration en utilisant une membrane avec un seuil de coupure de 300 kDa (Millipore PTMK300). La suspension virale inactivée est conservée à +5°C.

### Exemple 17 : Préparation du vaccin sous forme d'émulsion à base d'huile minérale.

Le vaccin est préparé selon la formule suivante :
- suspension de circovirus porcin inactivé : 250 ml
- Montanide® ISA 70 (SEPPIC): 750 ml

La phase aqueuse et la phase huileuse sont stérilisées séparément par filtration. L'émulsion est préparée par mélange et homogénéisation des ingrédients à l'aide d'un émulseur à turbine Silverson.

Une dose de vaccin contient environ 10^{7,5} DICT50. Le volume d'une dose de vaccin est de 0,5 ml pour administration par voie intradermique, et de 2 ml pour administration par voie intramusculaire.

### Exemple 18 : Préparation du vaccin sous forme d'émulsion à base d'huile métabolisabie.

Le vaccin est préparé selon la formule suivante :
- suspension de circovirus porcin inactivé : 200 ml
- Dehymuls HRE 7 (Henkel) : 60 ml
- Radia 7204 (Oleofina) : 740 ml

La phase aqueuse et la phase huileuse sont stérilisées séparément par filtration. L'émulsion est préparée par mélange et homogénéisation des ingrédients à l'aide d'un émulseur à turbine Silverson.

Une dose de vaccin contient environ 10^{7,5} DICT50. Le volume d'une dose de vaccin est de 2 ml pour administration par voie intramusculaire.

### Exemple 19 : Résultats d'immunofluorescence indirecte vis-à-vis des souches de virus PCV US et française et du contaminant PK/15 avec un sérum hyperimmun (PCV-T), un panel d'anticorps monoclonaux F99, préparés à partir de PK/15 et un sérum hyperimmun préparé à partir de la souche canadienne (PCV-C)

| VIRUS | | | |
|---|---|---|---|
| | PK/15 | USA | France |
| PCV-T antiserum | ≥ 6 400 | 200 | 800 |
| PCV-C antiserum | 200 | ≥ 6,400 | ≥ 6,400 |
| F99 1H4 | ≥ 10 000 | < 100 | 100 |
| F99 4B10 | ≥ 10 000 | < 100 | < 100 |
| F99 2B7 | ≥ 10 000 | 100 | < 100 |
| F99 2E12 | ≥ 10 000 | < 100 | < 100 |
| F99 1C9 | ≥ 10 000 | < 100 | 100 |
| F99 2E1 | ≥ 10 000 | < 100 | < 100 |
| F99 1H4 | ≥ 10 000 | 100 | < 100 |
| * inverse de la dernière dilution du sérum ou de l'anticorps monoclonal qui donne une réaction positive en immunofluorescence indirecte. | | | |

## Revendications

1. Vaccin induisant une réponse immunitaire contre le circovirus porcin de type II (PCV type II), comprenant un circovirus porcin de type II responsable du syndrome de dépérissement généralisé de post-sevrage (PMWS), sous forme inactivée.

2. Vaccin selon la revendication 1, **caractérisé en ce que** le PCV type II est susceptible d'être isolé d'un échantillon physiologique ou d'un prélèvement tissulaire d'un porc malade présentant le syndrome PMWS.

3. Vaccin selon la revendication 1, **caractérisé en ce que** le PCV type II est issu de l'une des souches déposées auprès de l'ECACC sous les références:
- V97 100219
- V97 100218
- V97 100217
- V98 011608
- V98 011609.

4. Vaccin selon l'une quelconque des revendications 1 à 3, **caractérisé en ce qu'**il comprend de 10⁶ à 10⁸ TCID₅₀ de PCV type II.

5. Vaccin selon l'une quelconque des revendications 1 à 4, **caractérisé en ce qu'**il comprend en outre un adjuvant.

6. Vaccin selon la revendication 5, **caractérisé en ce qu'**il comprend de l'hydroxyde d'alumine, de la saponine, de l'Avridine®, du DDA ou du polyphosphazene.

7. Vaccin selon l'une quelconque des revendications 1 à 4, **caractérisé en ce qu'**il est formulé sous forme d'une émulsion.

8. Vaccin selon la revendication 7, **caractérisé en ce qu'**il est formulé sous forme d'une émulsion huite-dans-l'eau.

9. Vaccin selon la revendication 7, **caractérisé en ce qu'**il est formulé sous forme d'une émulsion eau-dans-l'huile.

10. Vaccin selon l'une quelconque des revendications 1 à 9, **caractérisé en ce que** le PCV type II a été inactivé par un agent chimique, notamment un agent chimique choisi parmi: formaldéhyde, paraformaldéhyde, β-propiolactone, éthylèneimine ou un dérivé de l'éthylèneimine.

11. Vaccin selon l'une quelconque des revendications 1 à 10, **caractérisé en ce que** le PCV type II a été propagé sur une lignée cellulaire, de préférence de porc.

12. Vaccin selon la revendication 11, **caractérisé en ce qu'**il s'agit de cellules PK/15.

13. Vaccin selon l'une quelconque des revendications 1 à 12, **caractérisé en ce qu'**il comprend au moins une autre valence choisie parmi Porcine Reproductory and Respiratory Syndrome (PRRS), Mycoplasma hyopneumoniae, Actinobacillus pleuropneumoniae, E. coli, rhinite atrophique, maladie d'Aujeszky, peste porcine classique, grippe porcine.

14. Vaccin selon la revendication 13, **caractérisé en ce qu'**il comprend une valence PRRS et/ou une valence Mycoplasma hyopneumoniae.

## Patentansprüche

1. Impfstoff zum Hervorrufen einer Immunantwort gegen das Schweinecircovirus vom Typ II (Typ-II-PCV), mit einem Schweinecircovirus des Typs II, das für das verallgemeinerte Verfallssyndrom nach Entwöhnung (PMWS) verantwortlich ist, in desaktivierter Form.

2. Impfstoff nach Anspruch 1, **dadurch gekennzeichnet, dass** das Typ-II-PCV aus einer physiologischen Probe oder einer Gewebeentnahme eines kranken Schweines, das das PMWS-Syndrom zeigt, isolierbar ist.

3. Impfstoff nach Anspruch 1, **dadurch gekennzeichnet, dass** das Typ-II-PCV aus einem der Stämme hervorgeht, die beim ECACC unter folgenden Aktenzeichen hinterlegt sind:
- V97 100219
- V97 100218
- V97 100217
- V98 011608
- V98 011609.

4. Impfstoff nach einem der Ansprüche 1 bis 3, **dadurch gekennzeichnet, dass** er 10⁶ bis 10⁸ TCID₅₀ des Typ-II-PCV aufweist.

5. Impfstoff nach einem der Ansprüche 1 bis 4, **dadurch gekennzeichnet, dass** er außerdem einen Hilfsstoff aufweist.

6. Impfstoff nach Anspruch 5, **dadurch gekennzeichnet, dass** er Aluminiumhydroxid, Saponin, Avridin®, DDA oder Polyphosphazen aufweist.

7. Impfstoff nach einem der Ansprüche 1 bis 4, **dadurch gekennzeichnet, dass** er als Emulsion zubereitet ist.

8. Impfstoff nach Anspruch 7, **dadurch gekennzeichnet, dass** er als Emulsion von Öl in Wasser zubereitet ist.

9. Impfstoff nach Anspruch 7, **dadurch gekennzeichnet, dass** er als Emulsion von Wasser in Öl zubereitet ist.

10. Impfstoff nach einem der Ansprüche 1 bis 9, **dadurch gekennzeichnet, dass** das Typ-II-PCV durch ein chemisches Reaktionsmittel desaktiviert wurde, und zwar insbesondere durch ein Reaktionsmittel, das aus Formaldehyd, Paraformaldehyd, β-Propiolacton, Ethylenimin oder einem Ethyleniminabkömmling gewählt ist.

11. Impfstoff nach einem der Ansprüche 1 bis 10, **dadurch gekennzeichnet, dass** das Typ-II-PCV auf einer Zelllinie, vorzugsweise einer Schweinezelllinie, verbreitet wurde.

12. Impfstoff nach Anspruch 11, **dadurch gekennzeichnet, dass** es sich um PK/15-Zellen handelt.

13. Impfstoff nach einem der Ansprüche 1 bis 12, **dadurch gekennzeichnet, dass** er wenigstens eine weitere Wertigkeit umfasst, die aus dem Reproduktions- und Atmungssyndrom der Schweine (PRRS: Porcine Reproductory and Respiratory Syndrome), Mycoplasma hyopneumoniae, Actinobacillus pleuropneunomiae, E. coli, atrophische Nasenentzündung, Aujeszkische Krankheit, klassische Schweinepest, Schweinegrippe gewählt ist.

14. Impfstoff nach Anspruch 13, **dadurch gekennzeichnet, dass** er eine Wertigkeit gegen PRRS und/oder eine Wertigkeit gegen Mycoplasma hyopneumoniae umfasst.

## Claims

1. Vaccine inducing an immune response to type II porcine circovirus (PCV type II), comprising a type II porcine circovirus responsible for Postweaning Multisystemic Wasting Syndrome (PMWS), in inactivated form.

2. Vaccine according to claim 1, **characterised in that** the PCV type II is capable of being isolated from a physiological sample or a tissue sample from a sick pig suffering from PMWS.

3. Vaccine according to claim 1, **characterised in that** the PCV type II has been obtained from one of the strains deposited at the ECACC under the references:
- V97 100219
- V97 100218
- V97 100217
- V98 011608
- V98 011609.

4. Vaccine according to any one of claims 1 to 3, **characterised in that** it comprises from 10⁶ to 10⁸ TCID₅₀ of PCV type II.

5. Vaccine according to any one of claims 1 to 4, **characterised in that** it further comprises an adjuvant.

6. Vaccine according to claim 5, **characterised in that** it comprises aluminium hydroxide, saponin, Avridine®, DDA or polyphosphazene.

7. Vaccine according to any one of claims 1 to 4, **characterised in that** it is formulated in the form of an emulsion.

8. Vaccine according to claim 7, **characterised in that** it is formulated in the form of an oil-in-water emulsion.

9. Vaccine according to claim 7, **characterised in that** it is formulated in the form of a water-in-oil emulsion.

10. Vaccine according to any one of claims 1 to 9, **characterised in that** the PCV type II has been inactivated by a chemical agent, notably a chemical agent selected from among: formaldehyde, paraformaldehyde, β-propiolactone, ethyleneimine or an ethyleneimine derivative.

11. Vaccine according to any one of claims 1 to 10, **characterised in that** the PCV type II has been propagated on a cell line, preferably a pig cell line.

12. Vaccine according to claim 11, **characterised in that** the cells are PK/15 cells.

13. Vaccine according to any one of claims 1 to 12, **characterised in that** it comprises at least one other vector selected from Porcine Reproductory and Respiratory Syndrome (PARS), Mycoplasma hyopneumoniae, Actinobacillus pleuropneumoniae, E. coli, atrophic rhinitis, Aujeszky's disease, classic swine fever, swine influenza.

14. Vaccine according to claim 13, **characterised in that** it comprises a PRRS carrier and/or a Mycoplasma hyopneumoniae carrier.
